# EUROPÄISCHE PATENTSCHRIFT

(11) **EP 1 443 975 B1**
(45) Veröffentlichungstag und Bekanntmachung des Hinweises auf die Patenterteilung: **29.06.2005**
(21) Anmeldenummer: 02782891.2
(22) Anmeldetag: 11.10.2002
(51) Int. Cl.: A61L 2/00, A61L 2/16

(54) **VERFAHREN UND KIT ZUM MASCHINELLEN REINIGEN UND DESINFIZIEREN VON MEDIZINISCHEN INSTRUMENTEN**
METHOD AND KIT FOR MECHANICALLY CLEANING AND STERILIZING MEDICAL INSTRUMENTS
PROCEDE ET KIT DE NETTOYAGE ET DE DESINFECTION PAR MACHINE D'INSTRUMENTS MEDICAUX

(30) Priorität: 09.11.2001 EP 01126781
(43) Veröffentlichungstag der Anmeldung: 11.08.2004
(73) Patentinhaber: Chemische Fabrik Dr. Weigert GmbH & Co. KG., 20539 Hamburg (DE)
(72) Erfinder: WAGEMANN, Wolfgang, 22967 Tremsbüttel (DE); KAMER, Markus, 21465 Reinbek (DE)
(74) Vertreter: Glawe, Delfs, Moll & Partner
(86) Internationale Anmeldenummer: PCT/EP2002/011421
(87) Internationale Veröffentlichungsnummer: WO 2003/039605

(56) Entgegenhaltungen:
- EP-A- 0 884 115
- WO-A-85/04107
- WO-A-96/10916
- US-A- 5 324 477
- US-A- 6 015 529

## Beschreibung

Die Erfindung betrifft ein Verfahren zum maschinellen Reinigen und Desinfizieren von medizinischen sowie chirurgischen Instrumenten und Apparaten wie beispielsweise Endoskopen und/oder deren Teile, mit den folgenden Schritten:
a) Reinigen der Instrumente und Apparate mit einer wäßrigen Reinigungslösung,
b) Desinfizieren der Instrumente und Apparate mit einer wäßrigen Desinfektionsmittellösung.

Gegenstand der Erfindung ist ferner ein Kit zur Durchführung des Verfahrens.

In der medizinischen Therapie und Diagnostik werden eine Vielzahl von Eingriffen mit Hilfe von Endoskopen durchgeführt. Diese Endoskope werden bei ihrem Einsatz verunreinigt und mit vielerlei Mikroorganismen infiziert.

Die Reinigung und Desinfektion benutzter Endoskope ist schwierig, da Endoskope, insbesondere moderne Glasfiberendoskope, Oberflächen aus einer Vielzahl unterschiedlicher Materialien aufweisen, die teilweise temperaturempfindlich und teilweise korrosionsanfällig sind. Problematisch ist auch das vollständige Reinigen und Desinfizieren der Vielzahl von Hohlräumen in Endoskopen, insbesondere der im Inneren vorhandenen englumigen Kanäle.

Ein eingangs genanntes Verfahren ist aus EP-A-0 268 227 bekannt. Die wäßrige Desinfektionsmittellösung enthält Glutaraldehyd als Desinfektionsmittel. Viele Aldehyde sind gesundheitlich bedenklich und können nur mit besonderen Vorsichtsmaßnahmen gehandhabt werden.

WO-A-96/10916 offenbart die Desinfektion von medizinischen Instrumenten mit Chlordioxid. Es wird dort aus einer NatriumChloritlösung einerseits und einer Säure andererseits eine chlordioxidhaltige Stammlösung angesetzt, die nach Verdünnung zur Desinfektion verwendet wird.

Der Erfindung liegt die Aufgabe zugrunde, ein Verfahren der eingangs genannten Art zu schaffen, mit dessen Hilfe ein einfaches, schnelles und wirkungsvolles maschinelles Reinigen und Desinfizieren von medizinischen Instrumenten und Apparaten möglich ist.

Das erfindungsgemäße Verfahren ist dadurch gekennzeichnet, daß während des Desinfektionsschrittes als Desinfektionsmittel Chlordioxid aus einem Chlorit (beispielsweise einem Alkalichlorit wie Natriumchlorit) in situ freigesetzt wird.

Zunächst seien einige im Rahmen der Erfindung verwendete Begriffe erläutert.

Medizinische/chirurgische Instrumente und Apparate sind sämtliche Vorrichtungen oder Teile von Vorrichtungen, die in der Medizin, in der Chirurgie oder im Krankenhausbereich eingesetzt werden und einer maschinellen Reinigung grundsätzlich zugänglich sind.

Der Begriff "Endoskope und/oder deren Teile" umfaßt alle im Bereich der Diagnostik, Therapie und Chirurgie eingesetzten flexiblen und starren Endoskope, die bei bestimmungsgemäßem Gebrauch verunreinigt bzw. kontaminiert werden können, sowie Teile dieser Instrumente bzw. Apparate.

Das erfindungsgemäße Verfahren ist verwendbar für starre Endoskope (bspw. Laparoskope für die minimalinvasive Chirurgie). Besonders vorteilhaft ist dieses Verfahren bei flexiblen Endoskopen, bspw. Glasfiber-Endoskopen, die in ihren flexiblen Bereichen häufig englumige und schwer zu reinigende Hohlräume aufweisen. Flexibel im Sinne der Erfindung sind Endoskope dann, wenn sie Teile oder Bereiche aufweisen, die sich bei bestimmungsgemäßem Gebrauch verbiegen oder verformen können.

Das Reinigen und Desinfizieren erfolgt erfindungsgemäß maschinell. Dies bedeutet, daß ein Eingriff von Hand beim Reinigen und/oder Desinfizieren nicht erforderlich ist. Insbesondere eignet sich das erfindungsgemäße Verfahren zur Anwendung in üblichen Spülmaschinen für medizinische Instrumente und Apparate, in denen der Reinigungs- und Desinfektionsschritt nacheinander in dem gleichen Spültank ablaufen kann. Diese Spülmaschinen weisen bevorzugt Einrichtungen bzw. Anschlüsse für die Endoskope auf, so daß die jeweils in der Maschine umgewälzte Lösung auch durch die Kanäle der Endoskope gepumpt wird und diese so von innen reinigt, desinfiziert oder spült.

Die Begriffe Reinigungslösung bzw. Desinfektionsmittellösung bezeichnen die anwendungsfertigen entsprechenden wäßrigen Lösungen, die in der Regel durch Verdünnen von Konzentraten mit vorzugsweise enthärtetem oder vollentsalztem Wasser hergestellt werden.

Erfindungsgemäß wird während des Desinfektionsschrittes Chlordioxid aus einem Chlorit in situ freigesetzt. Dies bedeutet, daß das Chlordioxid in der wässrigen Desinfektionsmittellösung durch eine chemische Reaktion freigesetzt wird. Demgegenüber wird im Stand der Technik das Chlordioxid (in der Regel in einem separaten sog. Chlordioxidgenerator) durch Zusammengeben von konzentrierten Chlorit- und Säurelösungen hergestellt, diese sog. Chlordioxidstammlösung wird dann verdünnt und erst anschließend mit den zu desinfizierenden Teilen in Berührung gebracht. In Getränkeindustrie 7/89, S. 60:0, ist beispielsweise ein unter dem Namen Bellozon geläufiges Verfahren beschrieben, bei dem als Ausgangschemikalien zur Chlordioxidherstellung 9%ige Salzsäure und eine 7,5%ige NatriumChloritlösung verwendet werden. Diese Chemikalien werden in einem separaten Generator zur Reaktion gebracht, die erhaltene Chlordioxidlösung wird anschließend als Desinfektionsmittel verwendet.

Die Erfindung hat erkannt, daß sich diese aufwendige Herstellung von Chlordioxid auf Vorrat vor der eigentlichen Desinfektion vermeiden läßt, indem man vergleichsweise geringe Konzentrationen von Chlorit und Säure in der fertigen Desinfektionsmittellösung reagieren läßt. Erfindungsgemäß läßt sich in der Desinfektionsmittellösung ohne weiteres eine mikrobiozide Chlordioxidkonzentration von 1 bis 500 ppm, vorzugsweise 2 bis 200 ppm, weiter vorzugsweise 2 bis 100 ppm, weiter vorzugsweise 5 bis 100 ppm, weiter vorzugsweise 5 bis 50 ppm, erreichen.

Die Chloritkonzentration (berechnet als Natriumchlorit) in der Desinfektionsmittellösung beträgt vorzugsweise 0,01 bis 1 Gew.-%, weiter vorzugsweise 0,02 bis 0,5 Gew.-%, weiter vorzugsweise 0,05 bis 0,3 Gew.-%.

Nach Zugabe des sauren Aktivators wird sich in der Regel ein pH-Wert von 1 bis 6, vorzugsweise 2 bis 5, insbesondere etwa 2 bis 4 einstellen. Die Reinigerlösung ist vorzugsweise nicht gepuffert, um die pH-Änderung durch Zugabe des sauren Aktivators zu erleichtern.

Die zur Erzeugung von Chlordioxid in situ erforderliche Säurekonzentration (berechnet als Salzsäure) kann beispielsweise 0,01 bis 0,5, Gew.-%, weiter vorzugsweise 0,02 bis 0,2 Gew.-%, weiter vorzugsweise 0,02 bis 0,1 Gew.-% betragen. Größere Säuremengen zur Einstellung des erforderlichen pH-Wertes für die Chlordioxidfreisetzung können insbesondere dann erforderlich sein, wenn die Desinfektionsmittellösung Puffer wie beispielsweise Phosphate enthält. Erfindungsgemäß kann als sog. saurer Aktivator Schwefelsäure oder Salzsäure verwendet werden, jedoch wird man in der Regel weniger korrosive Säuren wie beispielsweise Phosphorsäure, Borsäure oder organische Säuren wie Ameisensäure, Essigsäure, Oxalsäure, Malonsäure, Bernsteinsäure, Adipinsäure, Glycolsäure, Milchsäure, Gluconsäure, Weinsäure, Apfelsäure, Maleinsäure, Citronensäure, Ascorbinsäure, Amidosulfonsäure, Sorbinsäure oder dergleichen verwenden.

Im Rahmen der Erfindung kann am Ende des Reinigungsschrittes a) die wässrige Reinigungslösung verworfen und zu Beginn des Schrittes b) eine wässrige Desinfektionsmittellösung neu angesetzt bzw. eindosiert werden, in der dann in situ durch Zugabe eines sauren Aktivators Chlordioxid hergestellt wird.

Besonders bevorzugt ist es jedoch im Rahmen der Erfindung, wenn die in Schritt a) verwendete wässrige Reinigungslösung von vornherein Chlorit enthält. Nach Abschluß des Reinigungsvorgangs beläßt man diese Reinigungslösung dann als Desinfektionsmittellösung in der Spülmaschine und dosiert einen sauren Aktivator hinzu, so daß die Chlordioxidfreisetzung in situ beginnt. Bei Bedarf kann zu Beginn des Desinfektionsschrittes noch einmal chlorithaltiger Reiniger zudosiert werden, bevor, während oder nachdem saurer Aktivator zudosiert wird. Dieser saure Aktivator kann eine verhältnismäßig hochkonzentrierte Säurelösung sein, bei der Verwendung von Salzsäure kann beispielsweise eine 14%ige HCl-Lösung verwendet werden. Auf diese Weise wird die wässrige Reinigungslösung noch einmal als Desinfektionsmittellösung verwendet und zudem im Desinfektionsschritt gleich mit entkeimt, so daß sie nach Abschluß des Desinfektionsschrittes ohne weiteres in die Kanalisation abgelassen werden kann. Durch diese Verfahrensvariante kann die erforderliche Gesamtdauer zur Reinigung/Desinfektion wesentlich verkürzt werden.

Die beschriebene Variante des erfindungsgemäßen Verfahrens ist auch deswegen besonders vorteilhaft, da sie ohne weiteres in üblichen Spülmaschinen zur Reinigung und Desinfektion medizinischer Instrumente und Apparate eingesetzt werden kann. Solche Spülmaschinen besitzen in der Regel zwei voneinander getrennte Dosierpumpen, von denen eine üblicherweise zur Dosierung eines Reinigungsmittelkonzentrats im Reinigungsschritt und die zweite zur Dosierung eines Desinfektionsmittelkonzentrats im Desinfektionsschritt verwendet wird. Erfindungsgemäß kann zu Beginn des Reinigungsschrittes von einer der Pumpen ein Reinigungsmittelkonzentrat zur Herstellung der Reinigungslösung zudosiert werden; dieses Konzentrat enthält dann gleich Chlorit, wie beispielsweise Natrium- oder Kalium-Chlorit in der erforderlichen Menge.

Nach Abschluß des Reinigungsschrittes wird zur Einleitung des Desinfektionsschrittes lediglich von der zweiten Dosierpumpe eine Säurelösung als saurer Aktivator hinzudosiert, so daß in der benutzten Reinigungsmittellösung (die jetzt Desinfektionsmittellösung ist) in situ die Freisetzung von Chlordioxid beginnt.

Bei der beschriebenen Verfahrensvariante wird die Reinigerlösung in der Regel einen pH-Wert von 5 bis 10, vorzugsweise etwa 6 bis 9 aufweisen und somit im neutralen bis leicht alkalischen Bereich liegen. Es können übliche Reinigerbestandteile wie Tenside, Enzyme, Komplexbildner, Phosphate, Korrosionsinhibitoren usw. enthalten sein.

Die Temperatur während des Desinfizierens kann zwischen Raumtemperatur und nahe der Siedetemperatur der verwendeten Lösungen liegen. Bei der Reinigung empfindlicher Instrumente wie beispielsweise Endoskope oder deren Teile, Instrumente aus dem Bereich der Anästhesie oder dergleichen wird man in der Regel Temperaturen von 20 bis 60°C, insbesondere etwa 40° C, bevorzugen.

Eine übliche Zeitdauer des Reinigungs- und Desinfektionsschrittes beträgt jeweils 1 Minute bis 1 Stunde, bevorzugt etwa 2 bis 30 Minuten, weiter vorzugsweise etwa 2 bis 15 Minuten. Übliche Werte sind eine Zeitdauer von 3 bis 5 Minuten für den Reinigungsschritt und 5 bis 15, insbesondere etwa 5 bis 10 Minuten für den Desinfektionsschritt. Die Dauer des Desinfektionsschrittes muß ausreichend gewählt werden, daß sich durch die in situ-Freisetzung von Chlordioxid eine wirksame mikrobiozide Konzentration einstellen kann. Die erforderliche Dauer des Desinfektionsschrittes hängt damit insbesondere auch von der eingesetzten Chlorit- und Säurekonzentration sowie der Temperatur der Lösung ab.

Die Aufzählung der erfindungsgemäßen Schritte im Hauptanspruch ist nicht abschließend, es können übliche Vorreinigungsschritte, Zwischen- und insbesondere Schlußspülungen vorgesehen sein. Üblich ist insbesondere eine Schlußspülung mit vollentsalztem Wasser.

Die erfindungsgemäß verwendeten Reinigungs- und Desinfektionsmittellösungen können aus Konzentraten mit enthärtetem bzw. vollensalztem Wasser angesetzt werden, jedoch kann auch ein Ansetzen mit üblichem Stadtwasser erfolgen.

Ein Ausführungsbeispiel der Erfindung wird im folgenden beschrieben.

Es werden ein Reinigerkonzentrat (Komponente A) und ein saures Aktivatorkonzentrat (Komponente B) wie folgt angesetzt:
Komponente A:
   10% Natriumchloritlösung, 30%ig
   40% Kaliumtripolyphosphatlösung, 50%ig
   50% vollentsalztes Wasser
Komponente B:
   12%ige Salzsäure

   In einer üblichen Spülmaschine für medizinische Instrumente wurden verschmutzte Instrumente für eine Zeitdauer von 15 min mit einer 1%igen wässrigen Lösung der Komponente A bei 50°C gereinigt.
   Nach Abschluß des Reinigungsvorgangs wurde der Desinfektionsvorgang eingeleitet durch Zugabe von 1 Gew.-% (bezogen auf die Reinigungsflotte) der sauren Aktivatorkomponente B. Durch diese Zugabe wurde die Reinigungslösung in eine Desinfektionsmittellösung im Sinne der Erfindung überführt. Nach der Zugabe sank der pH-Wert der Desinfektionsmittellösung auf etwa 2 und es setzte eine Chlordioxidbildung ein. Die Desinfektion wurde über einen Zeitraum von 10 min bei einer Temperatur von 50°C durchgeführt. Nach diesem Zeitraum hatte sich in der Desinfektionsmittellösung ein Chlordioxidgehalt von etwa 20 ppm eingestellt.
   Nach Abschluß des Desinfektionsschrittes wurde die sterile Desinfektionsmittellösung abgelassen und die Instrumente mit vollentsalztem Wasser nachgespült.

## Patentansprüche

1. Verfahren zum maschinellen Reinigen und Desinfizieren von medizinischen sowie chirurgischen Instrumenten und Apparaten, mit den folgenden Schritten:
a) Reinigen der Instrumente und Apparate mit einer wäßrigen Reinigungslösung,
b) Desinfizieren der Instrumente und Apparate mit einer wäßrigen Desinfektionsmittellösung,
**dadurch gekennzeichnet, daß** während des Desinfektionsschrittes als Desinfektionsmittel Chlordioxid aus Chlorit und einem sauren Aktivator in situ freigesetzt wird.

2. Verfahren nach Anspruch 1, **dadurch gekennzeichnet, daß** die Chloritkonzentration (berechnet als Natriumchlorit) in der Desinfektionsmittellösung vorzugsweise 0,01 bis 1 Gew.-%, weiter vorzugsweise 0,02 bis 0,5 Gew.-%, weiter vorzugsweise 0,05 bis 0,3 Gew.-% beträgt.

3. Verfahren nach Anspruch 1 oder 2, **dadurch gekennzeichnet, daß** im Desinfektionsschritt nach Zugabe des sauren Aktivators sich ein pH-Wert von 1 bis 6, vorzugsweise 2 bis 5, weiter vorzugsweise von 2 bis 4 einstellt.

4. Verfahren nach Anspruch 1 bis 3, **dadurch gekennzeichnet, daß** in der Desinfektionsmittellösung eine Chlordioxidkonzentration von 1 bis 500 ppm, vorzugsweise 2 bis 200 ppm, weiter vorzugsweise 2 bis 100 ppm, weiter vorzugsweise 5 bis 100, weiter vorzugsweise 5 bis 50 ppm erreicht wird.

5. Verfahren nach einem der Ansprüche 1 bis 4, **dadurch gekennzeichnet, daß** in Schritt b) eine Chlorit enthaltende Lösung und ein saurer Aktivator getrennt zudosiert werden.

6. Verfahren nach einem der Ansprüche 1 bis 4, **dadurch gekennzeichnet, daß** die in Schritt a) zudosierte wäßrige Reinigungslösung Chlorit enthält und in Schritt b) ein saurer Aktivator zudosiert wird.

7. Verfahren nach einem der Ansprüche 1 bis 6, **dadurch gekennzeichnet, daß** die Dauer des Desinfektionsschrittes 1 min bis 1 Std., bevorzugt 2 bis 30 min, weiter vorzugsweise 2 bis 15 min, weiter vorzugsweise 5 bis 10 min beträgt.

8. Verfahren nach einem der Ansprüche 1 bis 7, wobei das medizinische Instrument bzw. der medizinische Apparat ein Endoskop und/oder dessen Teile sind.

9. Kit zur Durchführung des Verfahrens nach einem der Ansprüche 1 bis 8, **dadurch gekennzeichnet, daß** er enthält:
a) ein Reinigerkonzentrat,
b) ein Desinfektionskonzentrat, das Chlorit enthält,
c) ein saures Aktivatorkonzentrat.

10. Kit zur Durchführung des Verfahrens nach einem der Ansprüche 1 bis 8, **dadurch gekennzeichnet, daß** er enthält:
a) ein Reinigerkonzentrat, das Chlorit enthält,
b) ein saures Aktivatorkonzentrat.

## Claims

1. A method for mechanically cleaning and disinfecting medical and surgical instruments and apparatuses having the following steps:
a) cleaning the instruments and apparatuses with an aqueous cleaning solution,
b) disinfecting the instruments and apparatuses with an aqueous disinfectant solution,
**characterized in that**, during the disinfection step, as disinfectant, chlorine dioxide is released in situ from chlorite and an acid activator.

2. The method as claimed in claim 1, **characterized in that** the chlorite concentration (calculated as sodium chlorite) in the disinfectant solution is preferably 0.01 to 1% by weight, more preferably 0.02 to 0.5% by weight, more preferably 0.05 to 0.3% by weight.

3. The method as claimed in claim 1 or 2, **characterized in that**, in the disinfection step, after adding the acid activator, a pH of 1 to 6, preferably 2 to 5, more preferably 2 to 4, is established.

4. The method as claimed in claims 1 to 3, **characterized in that**, in the disinfectant solution, a chlorine dioxide concentration is achieved of 1 to 500 ppm, preferably 2 to 200 ppm, more preferably 2 to 100 ppm, more preferably 5 to 100 ppm, more preferably 5 to 50 ppm.

5. The method as claimed in one of claims 1 to 4, **characterized in that**, in step b), a chlorite-containing solution and an acid activator are added separately.

6. The method as claimed in one of claims 1 to 4, **characterized in that** the aqueous cleaning solution added in step a) comprises chlorite and, in step b), an acid activator is added.

7. The method as claimed in one of claims 1 to 6, **characterized in that** the period of the disinfection step is 1 min to 1 hour, preferably 2 to 30 min, more preferably 2 to 15 min, more preferably 5 to 10 min.

8. The method as claimed in one of claims 1 to 7, the medical instrument or the medical apparatus being an endoscope and/or parts thereof.

9. A kit for carrying out the method as claimed in one of claims 1 to 8, **characterized in that** it contains:
a) a cleaner concentrate,
b) a disinfectant concentrate which comprises chlorite,
c) an acid activator concentrate.

10. A kit for carrying out the method as claimed in one of claims 1 to 8, **characterized in that** it comprises:
a) a cleaner concentrate, which comprises chlorite,
b) an acid activator concentrate.

## Revendications

1. Procédé de nettoyage et de désinfection à la machine d'instruments et d'appareils médicaux ainsi que chirurgicaux comportant les étapes suivantes :
a) Nettoyage des instruments et appareils avec une solution nettoyante aqueuse,
b) Désinfection des instruments et appareils avec une solution désinfectante aqueuse,
**caractérisé en ce que**, pendant l'étape de désinfection, du dioxyde de chlore composé de chlorite et d'un activateur acide est diffusé *in situ* en tant que produit désinfectant.

2. Procédé selon la revendication 1, **caractérisé en ce que** la concentration de chlorite (calculée en tant que chlorure de sodium) est comprise, dans la solution désinfectante, de préférence entre 0,01 et 1 % en poids, de manière encore plus préférée entre 0,02 et 0,5 % en poids, de manière encore plus préférée entre 0,05 et 0,3 % en poids.

3. Procédé selon la revendication 1 ou 2, **caractérisé en ce que**, dans l'étape de désinfection, après ajout de l'activateur acide, il apparaît une valeur de PH comprise entre 1 et 6, de préférence entre 2 et 5, de manière encore plus préférée entre 2 et 4.

4. Procédé selon la revendication 1 à 3, **caractérisé en ce que**, dans la solution de désinfection, on obtient une concentration en dioxyde de chlore allant de 1 à 500 ppm, de préférence de 2 à 200 ppm, de manière encore plus préférée de 2 à 100 ppm, de manière encore plus préférée de 5 à 100 ppm, de manière encore plus préférée de 5 à 50 ppm.

5. Procédé selon l'une quelconque des revendications 1 à 4, **caractérisé en ce qu'**à l'étape b) une solution contenant du chlorite et un activateur acide sont ajoutés séparément.

6. Procédé selon l'une quelconque des revendications 1 à 4, **caractérisé en ce que** la solution nettoyante aqueuse ajoutée à l'étape a) contient du chlorite et un activateur acide est ajouté à l'étape b).

7. Procédé selon l'une quelconque des revendications 1 à 6, **caractérisé en ce que** la durée de l'étape de désinfection est comprise entre 1 minute et 1 heure, de préférence entre 2 et 30 min, de manière encore plus préférée entre 2 et 15 min, de manière encore plus préférée entre 5 et 10 min.

8. Procédé selon l'une quelconque des revendications 1 à 7, l'instrument médical ou l'appareil médical étant un endoscope et/ou des parties de celui-ci.

9. Kit de réalisation du procédé selon l'une quelconque des revendications 1 à 8, **caractérisé en ce qu'**il contient :
a) un concentré de produit nettoyant,
b) un concentré de produit désinfectant qui contient du chlorite,
c) un concentré d'activateur acide.

10. Kit de réalisation du procédé selon l'une quelconque des revendications 1 à 8, **caractérisé en ce qu'**il contient :
a) un concentré de nettoyant qui contient du chlorite,
b) un concentré d'activateur acide.
